# EUROPEAN PATENT SPECIFICATION

(11) **EP 4 312 849 B1**
(45) Date of publication and mention of the grant of the patent: **06.05.2026**
(21) Application number: 22718614.5
(22) Date of filing: 25.03.2022
(51) Int. Cl.: A61B 18/24, A61B 18/26

(54) **SAFETY SENSOR FOR LASER INDUCED PRESSURE WAVE IN CONTRAST MEDIA WITHIN A CONTAINMENT CATHETER DURING VASCULAR THERAPY**
SICHERHEITSSENSOR FÜR LASERINDUZIERTE DRUCKWELLE IN KONTRASTMITTELN IN EINEM EINSCHLUSSKATHETER WÄHREND EINER GEFÄSSTHERAPIE
CAPTEUR DE SÉCURITÉ POUR ONDE DE PRESSION INDUITE PAR LASER DANS DES MILIEUX DE CONTRASTE À L'INTÉRIEUR D'UN CATHÉTER DE CONFINEMENT PENDANT UNE THÉRAPIE VASCULAIRE

(30) Priority: 30.03.2021 US 202163167667 P
(43) Date of publication of application: 07.02.2024
(73) Proprietor: Koninklijke Philips N.V., 5656 AG Eindhoven (NL)
(72) Inventor: CRONE, Tyler West, 5656 AG Eindhoven (NL)
(74) Representative: Philips Intellectual Property & Standards
(86) International application number: PCT/EP2022/057857
(87) International publication number: WO 2022/207471

(56) References cited:
- EP-A1- 3 342 457
- US-A1- 2013 072 753
- US-A1- 2020 046 429

## Description

### FIELD

The following relates generally to the catheter arts, thrombectomy arts, atherectomy arts, catheter safety arts, and related arts.

### BACKGROUND

Vascular therapy (e.g., thrombectomy, atherectomy, and so forth) devices are medical devices designed to remove or modify tissue or material from inside a diseased vessel (e.g., an artery, a vein, etc.). Intravascular devices attempt to remove this material without surgically opening the vessel. The mechanisms of intravascular removal can include simple suction, mechanical cutting, chemical dissolution, ablation through heat or light, maceration by mechanical or sonic energy, and so forth.

The mechanism of action in, for example, some types of laser atherectomy utilizing a laser for material removal, is referred to as ultraviolet (UV) photoablation. However, this approach is less effective for calcified deposits, due to lower absorption of the laser light (e.g., in the near ultraviolet, such as at 308 nm in some specific systems). The photoablation can be enhanced for the purposes of debulking tissues at the target sites, especially but not limited to calcified deposits. The use of contrast media in combination with a UV laser produces a Laser Induced Pressure Wave (LIPW). This involves laser energy absorption at a molecular level in the contrast media, release of that energy producing a vapor bubble which expands, followed by a rapid collapse. This mechanism is effective for the purpose of debulking plaque and/or calcified lesions within the blood vessel. In order to do this safely, the lasing in contrast protocol is performed with the laser and proximate absorbing contrast medium located within a containment catheter. US2020/0046429A1 discloses an example vascular therapy device able to provide a LIPW mode treatment using light emitters located within a cavity to transmit pulses of light energy into a liquid medium within the cavity.

The following discloses certain improvements.

### SUMMARY

The invention is defined in the appended claims.

In some embodiments disclosed herein, a vascular therapy device includes a catheter including at least one optical fiber with a light output aperture. A containment sheath has a sheath opening disposed at an end thereof. The catheter is disposed inside the containment sheath and movable relative to the containment sheath to extend the light output aperture beyond the sheath opening such that the light output aperture is outside of the containment sheath. A safety sensor is configured to detect whether the light output aperture is in a safe zone inside the containment sheath. The device is configured to operate in a saline mode in which saline flows through the containment sheath and in a contrast mode in which contrast medium flows through the containment sheath. The device further includes a device controller configured to automatically turn off a laser operating to inject light into the at least one optical fiber of the catheter in response to a safety interlock condition in which the device is operating in contrast mode and the safety sensor detects that the light output aperture is not in the safe zone inside the containment sheath.

One advantage resides in providing an intravascular device with a safeguard feature to prevent blood vessel damage.

Another advantage resides in providing an intravascular device with a safety sensor to prevent blood vessel damage.

Another advantage resides in providing an intravascular device with one or more sensors to determine when an ablating instrument operating under conditions that generate LIPW is within a containment sheath.

Another advantage resides in providing an intravascular device with one or more sensors to determine when an ablating instrument is within a containment sheath and a controller to cease supply of energy to an ablating instrument when the ablating instrument is outside of the containment sheath and is operating under conditions that generate LIPW.

A given embodiment may provide none, one, two, more, or all of the foregoing advantages, and/or may provide other advantages as will become apparent to one of ordinary skill in the art upon reading and understanding the present disclosure.

### BRIEF DESCRIPTION OF THE DRAWINGS

The disclosure may take form in various components and arrangements of components, and in various steps and arrangements of steps. The drawings are only for purposes of illustrating the preferred embodiments and are not to be construed as limiting the disclosure.
FIGURE 1 diagrammatically illustrates a vascular therapy device in accordance with the present disclosure.
FIGURE 2 diagrammatically illustrates another view of the device of FIGURE 1.
FIGURE 3 diagrammatically illustrates another view of the device of FIGURE 1.
FIGURE 4 diagrammatically illustrates another view of the device of FIGURE 1.
FIGURE 5 diagrammatically illustrates another embodiment of the device of FIGURE 1.
FIGURE 6 diagrammatically illustrates a method of performing a vascular therapy method using one of the devices of either FIGURE 1 or FIGURE 5.

### DETAILED DESCRIPTION

The following relates to an improved thrombectomy or atherectomy device. Such devices are used to remove atherosclerosis (i.e., plaque buildup) in an artery (atherectomy procedure) or to remove a thrombus or collagen buildup in a vein (thrombectomy procedure). These procedures are typically performed in the peripheral vascular system to treat blood vessels in a leg or arm, although the procedures may be applied to treat blood vessels in other anatomical areas.

The following more specifically relates to an improvement in a system for laser atherectomy or thrombectomy. This system includes a sheath (e.g., containment catheter; also referred to herein as a containment sheath) which surrounds an inner catheter (also called a laser catheter herein) that includes an optical fiber or fiber bundle with a light output aperture. In one typical workflow, in a first pass, the light output aperture is extended out of an open end of the containment catheter and a laser beam that is input to the optical fiber or fiber bundle directly ablates the lesion. This is usually done with saline flowing through the sheath, in what is referred to as a saline mode or saline infusion protocol.

However, the laser light is not strongly absorbed by calcium deposits, so that this first pass does not effectively remove calcium deposits. To address this problem, a second pass is performed as follows. The light output aperture is withdrawn back into the containment catheter, and the flow is switched from saline to a contrast medium, thus placing the device into contrast mode or a contrast infusion protocol. The laser beam is strongly absorbed by the contrast medium, breaking molecular bonds of the contrast medium, which releases the absorbed energy by producing an acoustic or sonic wave known as the Laser Induced Pressure Wave (LIPW) and resulting in the formation of a cavitation vapor bubbles. The LIPW passes through the containment catheter and interacts with calcium deposits to debulk the calcified plaque. It should be noted that this two-pass workflow is typical, other workflow sequences may be used. For example, the operator may switch back and forth between saline and contrast modes to gradually remove difficult deposits.

It is recognized herein that there may be a potential safety hazard with operation in the contrast mode, that is, under conditions that generate LIPW. Since the contrast medium flows out of the open end of the containment catheter, if the laser is operated when the light output aperture is extended out of the open end of the containment catheter then the contrast medium outside of the containment catheter absorbs the laser beam and LIPW is generated outside of the containment catheter, which can damage the blood vessel wall.

To address this problem, a safety sensor is provided as disclosed herein to detect when the light output aperture is extended outside of the containment catheter, and the system controller is configured to use information from the safety sensor to implement a safety interlock that prevents operation under conditions that generate LIPW when the light output aperture is not in a safe zone within the containment sheath. In the illustrative embodiment, the safety sensor includes a sheath end marker disposed on the containment catheter (i.e., sheath) at or near the open end of the containment catheter, and an aperture marker disposed on the laser catheter at or near the light output aperture. One of these markers is an active sensor which detects proximity of the other marker. Optionally, an LIPW zone marker is also disposed on the containment catheter at a location marking the boundary of the target LIPW operating zone within the containment catheter.

The controller is configured (e.g., by operative connection to receive readings from the safety sensor and by suitable programming) to implement the safety interlock feature, for example as follows. A state is maintained, which indicates whether the light output aperture is in within the containment catheter. The state is initial set to "inside" since the light output aperture is usually inside the containment catheter while the catheter is inserted into the vasculature and moved to the treatment site, and changes to "outside" in response the aperture marker crossing the sheath end marker during the start of the first pass in saline mode. Thereafter, the state changes back to "inside" in response to the aperture marker again crossing the sheath end marker during the retraction of the light output aperture back inside the containment catheter. The controller automatically turns off the laser if both of the following conditions are met: (1) the operating mode is contrast mode (e.g., suitably defined as any time the contrast medium is flowing) and (2) the state is "outside".

If the LIPW zone marker is also provided, then a zone state is similarly maintained which indicates whether the light output aperture is extended beyond the LIPW zone marker ("inside" zone) or further withdrawn into the containment catheter ("outside" zone). In this case, the laser is automatically turned off if: (1) the operating mode is contrast mode and (2) either the state is "outside" or the zone state is "outside". (In a variant embodiment, since the laser typically should not be used at all when the light output aperture is withdrawn further in than the safe zone for LIPW operation, the laser may be automatically turned off any time the state indicates the light output aperture is withdrawn further in than the safe zone).

The illustrative safety sensor employing one or two proximity sensors with components disposed on the laser catheter and containment sheath has advantages such as providing a compact design fully integrated with the device, and providing for relatively straightforward state-based data analysis to implement the safety interlock. However, other types of safety sensors are contemplated for monitoring the state (and optionally also the zone state), such as deriving the location of the light output aperture relative to the sheath opening from X-ray fluoroscopy images acquired during the laser atherectomy procedure (which is typically performed as an image-guided therapy (IGT)). Also, while laser atherectomy is typically performed in an artery, the disclosed devices and approaches are also suitably used in venous therapies, e.g., a thrombectomy.

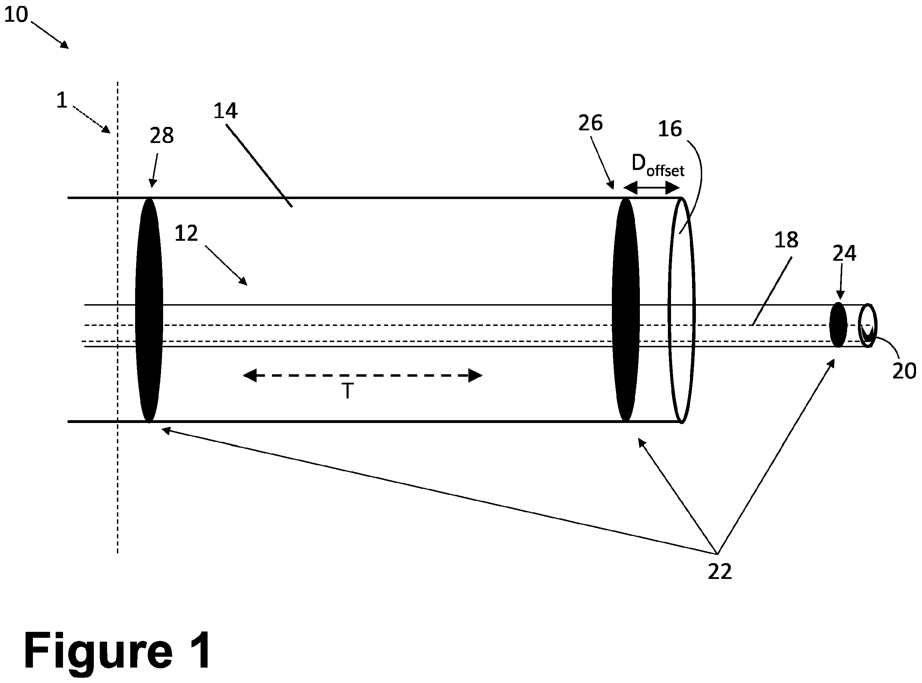

With reference to FIGURE 1, an illustrative vascular therapy (i.e., thrombectomy or atherectomy) device **10** is diagrammatically shown. The device **10** can be any suitable vascular therapy device (e.g., a Thor vascular therapy device, available from Koninklijke Philips N.V., Eindhoven, the Netherlands). As shown in FIGURE 1, the device **10** includes a catheter **12** surrounded by a containment catheter (also referred to herein as a containment sheath, or simply as a sheath) **14**. The illustrative containment catheter **14** surrounds the catheter **12** (i.e., the catheter **12** is an inner catheter relative to the containment sheath **14**). The catheter **12** is movable at least along a longitudinal translation direction **T** denoted in FIGURE 1 within the containment sheath **14**. In some embodiments, a distance between an outer diameter of the catheter **12** and an inner diameter of the containment sheath **14** can be, for example, 0.0021 inches, thereby providing a channel between the catheter **12** and the containment sheath **14** through which saline and/or a contrast medium can flow.

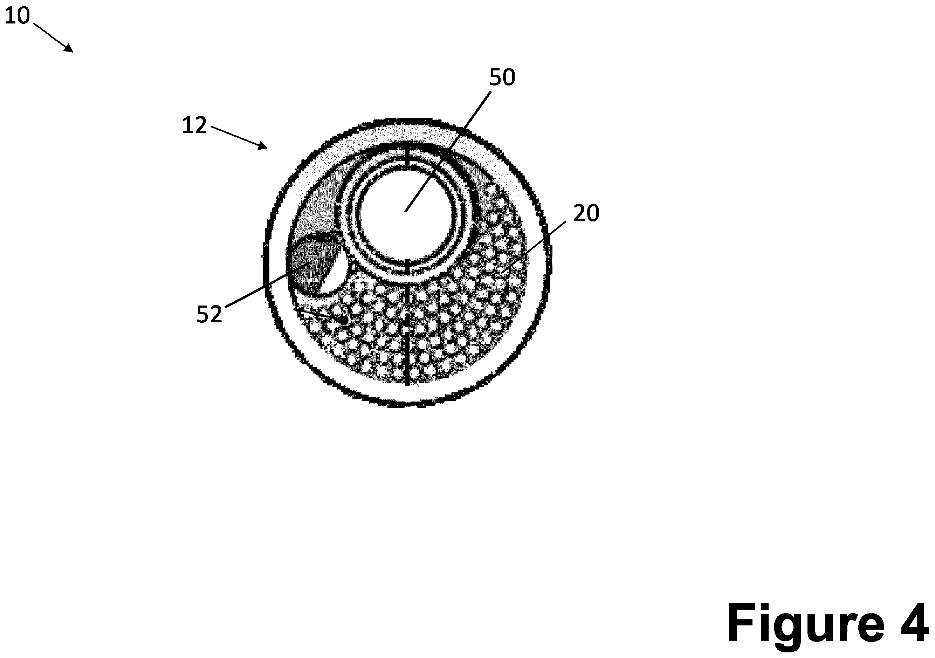

At one end of the containment sheath **14** is a sheath opening **16** through which the distal end of the catheter **12** can move into and out of the containment sheath **14**. The catheter **12** includes at least one optical fiber **18** (shown using dashed lines) with a light output aperture **20** that together comprise a tip of the catheter **12**. The illustrative light output aperture **20** is eccentrically positioned relative to the center axis of the catheter **12**, and is located at the very end of the catheter **12**. However, in other embodiments the light output aperture could have different geometry, e.g., could be centered on the center axis of the catheter **12** and/or could be located on a sidewall of the catheter **12** by way of a suitable bend in the optical fiber **18**. It will be appreciated that the optical fiber **18** may in some embodiments comprise an optical fiber bundle, as shown for example in FIGURE 4. The optical fiber(s) **18** are configured to carry light energy of a wavelength and intensity effective to perform UV photoablation or otherwise remove (i.e., ablate) a blood clot (e.g., a thrombus), plaque (e.g., atheroma), or other undesired material disposed on a blood vessel wall, by way of direct exposure of the material to the light (e.g., in saline mode) or by way of generating LIPW energy (e.g., in contrast mode), depending on the operational mode. The catheter **12** is disposed inside the containment sheath **14** and movable (at least movable back and forth along the translation direction **T**) relative to the containment sheath **14** to extend the light output aperture **20** beyond the sheath opening **18** such that the light output aperture **20** is outside of the containment sheath **14**.

FIGURE 1 also shows a safety sensor **22** configured to detect whether the light output aperture **20** is in a safe zone inside the containment sheath **14** (the safe zone is defined by lines **1** and **2** as shown in FIGURE 1). The safety sensor **22** is disposed on an end portion of the containment sheath **14**; although the safety sensor **22** may be disposed on any suitable portion of the containment sheath **10**. In some embodiments, the safety sensor **22** comprises a proximity sensor configured to detect passage events in which the light output aperture **20** passes through the sheath opening **16**. The passage events can include, for example, whether the light output aperture **20** has extended beyond the sheath opening **18**, whether the light output aperture **20** (once detected past the sheath opening **18**) has been withdrawn into the containment sheath **14**, and so forth. The proximity sensor **22** can be any suitable sensor to implement suitable detection methods, such as capacitive coupling, induction, infrared, photo-detection, ultrasonic, Hall effects, and so forth.

As shown in FIGURE 1, the proximity sensor comprises an aperture marker **24** disposed on the catheter **12**, and a sheath opening marker **26** disposed on a portion of the containment sheath 1**4**. One of the aperture marker **24** and the sheath opening marker **26** is a passive marker, and the other of the aperture marker **24** and the sheath opening marker **26** is an active marker configured to detect proximity of the passive marker. For example, in one embodiment the aperture marker **24** is a passive marker, and the sheath opening marker **26** is an active marker configured to detect proximity of the passive aperture marker. In another embodiment, the sheath opening marker **26** is a passive marker, and the aperture marker **24** is an active marker configured to detect proximity of the passive sheath opening marker. The passage events detected by the markers **24, 26** can include whether the aperture marker **24** has extended beyond the sheath opening marker **26**.

In some embodiments, as shown in FIGURE 1, the safety sensor **22** further includes a backside proximity sensor **28** configured to detect passage events in which the light output aperture **20** passes through an interior boundary of the safe zone of the containment sheath **14**. The interior boundary is located inside the containment sheath **14** and defined by line **1**.

The sheath opening marker **26** is preferably located close to the sheath opening **16**. In some embodiments, the sheath opening marker **26** is interior of the sheath opening **16** by a longitudinal offset distance **D_{offset}** that provides a safety margin chosen to account for the distance over which light exiting the light output aperture **20** may induce LIPW in the contrast medium. In some embodiments, the working distance of the light exiting the light output aperture **20** is small, e.g., on the order of microns or tens of microns, in which case the offset distance **D_{offset}** can be small or even zero (i.e., the marker **26** could be precisely at the sheath opening **16**).

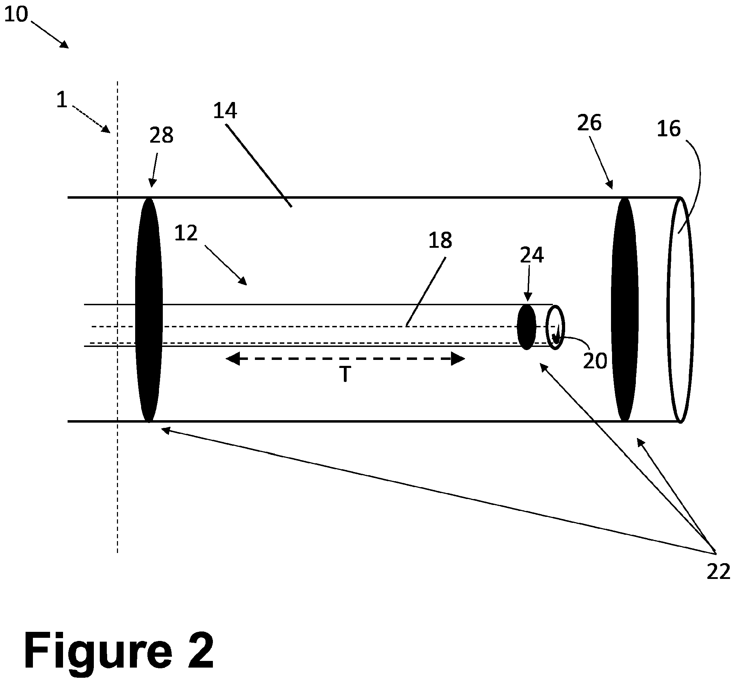

FIGURE 1 shows the light outlet aperture **20** and the aperture marker **24** outside of the sheath opening **18**. FIGURE 2 shows the light outlet aperture **20** and the aperture marker **24** inside of the sheath opening **18**.

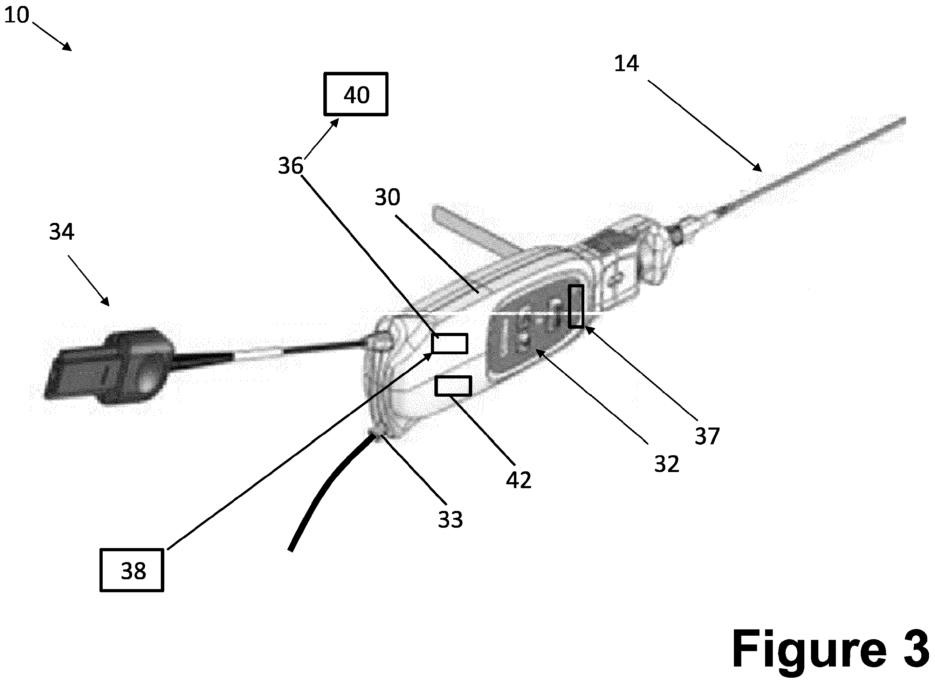

With reference now to FIGURE 3, and with continuing reference to FIGURES 1 and 2, the containment sheath **14** and the catheter **12** (not shown in FIGURE 3 but is disposed within the containment catheter **14** shown in FIGURE 3) are connected with a housing **30** of the device **10**. The device **10** also includes a motor drive unit (MDU) **32** configured to drive movement of the catheter **12** within a target area (e.g., an artery or a vein). The device **10** further includes a laser coupler **34** operatively connected with the at least one optical fiber **18** and an energy supply (not shown) to supply energy to the optical fiber(s) **18** to deliver ablation therapy to a lesion in the artery or vein. A fluid inlet **33** enables flow of a working fluid into the containment sheath **14**. For example, the working fluid may be switched between saline which is flowed when operating in saline mode, and contrast medium which is flowed when operating in contrast mode. The contrast medium should be absorbing for the light generated by the laser so that the contrast medium is energized to produce LIPW. Optionally, the contrast medium may also be absorbing for X-rays used in fluoroscopic imaging optionally performed to monitor the interventional vascular treatment procedure.

Within the housing **30** is an electronic processor **36** (e.g., a microprocessor) configured to control operation of the MDU **32** and/or the laser coupler **34**. In some examples, the electronic processor **36** can be an external computing device in electronic communication with the device **10**. The electronic processor **36** is configured or programmed to detect whether the light output aperture **20** is in the safe zone based at least on the passage events detected by the proximity sensor **22**. In some examples, when the device **10** includes the backside proximity sensor **28**, the electronic processor **34** is configured to detect whether the light output aperture **20** is in the safe zone further based on the passage events detected by the backside proximity sensor **28**. In some examples, a display device **37** can be included in the housing **30** and be operatively connected with the electronic processor **36**. The display device **37** can display information related to the ablation process, as described in more detail below. The electronic processor **36** also has information on whether the device is operating in contrast mode. In one approach, a user input (e.g., a switch) is used to enable the user to manually identify to the electronic processor **36** whether saline or contrast medium is flowing into the inlet **33**. In another approach, an optical sensor can be provided inside the housing **30** to automatically detect when contrast medium is flowing. For example, the optical sensor can be implemented as an LED/photodetector arrangement that detects the opacity of the fluid, with contrast medium being detected due to its higher opacity. In yet another approach, separate inlets are provided for the saline and contrast medium, and the housing **30** includes valves for switching between saline mode and contrast mode, and the electronic processor **36** controls those valves and hence the mode information is available to the electronic processor.

The electronic processor **36** can be configured or programmed to detect whether the light output aperture **20** is within the safe zone shown in FIGURE 1. In one example embodiment, one or more of the aperture marker **24** and the sheath opening marker **26** can be radiopaque. The electronic processor **36** is then configured to receive images from an interventional radiology imaging device **38** (shown diagrammatically in FIGURE 1 as a rectangle), such as a live fluoroscope, that depict the radiopaque aperture marker **24** and the radiopaque sheath opening marker **26**. In this embodiment, the radiopaque aperture marker **24** and the radiopaque sheath opening marker **26** do not detect passage events, and thus are separate from the safety or proximity sensor **22**. In this embodiment, the fluoroscope **38** comprises the safety sensor **22**. The electronic processor **36** then detects, from the received images, whether the light output aperture **20** is in the safe zone. (In another approach, an electronic processor of the fluoroscope **38** performs the image processing to detect whether the light output aperture **20** is in the safe zone, and sends an indication of whether the light output aperture **20** is in the safe zone to the processor **36** of the catheter MDU **30**). In some embodiments, the safe zone does not have an interior boundary within the containment sheath **14** (e.g., as defined in FIGURE 1 by line **1**). In such embodiments, an entire inside of the containment sheath **14** (or at least the entire portion of the inside of the containment sheath or at least the entire portion of the inside of the containment sheath **14** within which the light output aperture **20** could be located within which the light output aperture **20** could be located) is defined as the safe zone.

In another example embodiment, the electronic processor **36** is programmed to implement a state machine **40** indicative of one or more states of the light output aperture **20** relative to the sheath end **16**. For example, the electronic processor **36** is programmed to determine an inside state of the light output aperture **20** indicative of whether the light output aperture is inside of the containment sheath **14** (e.g., based on the detected passage events by the aperture marker **24** and the sheath opening marker **26** detecting the light output aperture **20** being within the containment sheath **14**). For example, if it is known that at time *t₀* the light output aperture **20** is inside the containment sheath **14** (e.g., if this is the state when the catheter is inserted into the blood vessel), then the state machine **40** can be set to an inside state of *Tip-State=inside safe zone.*

The electronic processor **36** is programmed to update a state from the inside state to an outside state after determining that the light output aperture **20** is outside of the containment sheath **14** (e.g., the sensors **24, 26** have detected a passage event that the light outpour aperture **20** has extended beyond the sheath opening **18**), i.e., *Tip-State=outside safe zone.* In some examples, the electronic processor **36** is programmed to further update the state of the light output aperture **20** from the outside state back to the inside state by determining whether the aperture marker **24** no longer extends past the sheath opening marker **26**. For example, at a time *t₁* that is later than the initial time *t₀*, the sensors **24, 26** can detect an event indicating the light output aperture **20** passes through the sheath opening **18**. This detected event can be assumed to be due to the light output aperture **20** being extended beyond the sheath opening **18**, so that the state machine **40** can be updated from *Tip-State=inside safe zone* to *Tip-State=outside safe zone.* In another example, at a time *t₂* later than *t₁* the sensors **24, 26** again detects an event indicating passage of the light output aperture **20** through the sheath opening **18**. This can be assumed to be a retraction operation and the state can be updated from *Tip-State=outside safe zone* to be switched back to *Tip-State=inside safe zone.* In other words, every time the sensors **24, 26** detect an event indicating the light output aperture **20** passes through the sheath opening **18**, the state can be updated to that the variable *Tip-state* can be switched from either *inside* to *outside* or from *outside* to *inside.*

In some embodiments, when the device **10** includes the backside proximity sensor **28**, the state machine **40** can include a third state *Tip-State=overwithdrawn.* Each time the backside proximity sensor **28** detects an event of the light output aperture **20**, the state machine **40** can be updated from *Tip-State=inside safe zone* to *Tip-State=overwithdrawn,* or from *Tip-State=overwithdrawn* to *Tip-State=inside safe zone.* It will be appreciated that there will never be a transition of the state machine **40** in which the state is updated from *Tip-State=overwithdrawn* to *Tip-State=outside safe zone* (or vice versa), as the light output aperture **20** would first have to pass through the sheath opening marker **26** which should switch the state machine **40** from *Tip-State=outside safe zone* to *Tip-State=inside safe zone.* In some embodiments, the state of light output aperture **20** can be displayed on the display device **37** (e.g., "inside", "open", "overwithdrawn", and so forth).

The device **10** is configured to operate in a saline mode in which saline flows through the containment sheath **14**, and in a contrast mode in which contrast medium flows through the containment catheter **14**. The saline is typically flowed during operation in which the light output at the aperture **20** directly ablates the lesion. In this saline mode, the aperture **20** is extended outside the sheath opening **16**. The contrast medium is used in contrast mode to provide light absorption and response effective to produce light induced pressure waves (LIPW) thereby providing a different removal mechanism, one that is particularly well suited for removing calcified deposits that have low absorption for the light. In this mode, the light output aperture **20** should be withdrawn into the safe zone inside the sheath **14**. In addition to tracking the state of the light output aperture **20** relative to the sheath opening **18** using the state machine **40**, the electronic processor **36** is programmed to also track whether the catheter **12** is in the saline mode or the contrast mode, for example by way of a manual input, an optical sensor, by direct control of valves controlling the saline and contrast medium flows, or so forth. If the electronic processor **36** determines that (i) the catheter **12** is in the contrast mode and (ii) the state of the light output aperture is in the outside state, then the electronic processor 36 is configured to control the laser coupler **34** to cease supplying optical energy to the at least one optical fiber **18** (i.e., so that the ablation process is halted).

In some embodiments, in lieu of the electronic processor **36** controlling the supply of energy to the optical fiber(s) **18,** the device **10** includes a device controller **42** that is configured to automatically turn off the laser coupler **34** operating to inject light into the at least one optical fiber **18** of the catheter in response to a safety interlock condition in which the device **10** is operating in contrast mode and the safety sensor **22** detects that the light output aperture **20** is not in the safe zone inside the containment sheath **14**. In another suitable approach, the laser itself may be turned off in response to the safety interlock condition. The safety interlock condition can include the electronic processor **36** determining that (i) the catheter **12** is in the contrast mode and (ii) the state of the light output aperture is in the outside state.

FIGURE 4 shows a front view of the device **10**. Specifically, FIGURE 4 shows an end view of the inner catheter **12** (i.e., looking "straight-on" at the light outlet aperture **20**). FIGURE 4 shows the spatial relationship between the light outlet aperture **20** (which in this example is the end of a fiber optical bundle), an optional guide wire lumen **50** through which a guide wire used for guiding the catheter extends, and an illustrative additional lumen **52**. If the aperture marker **24** is an active marker, then for example the additional lumen **52** may provide the conduit for electrical wires running to the active aperture marker **24**. In this nonlimiting illustrative embodiment, the aperture **20** is arranged eccentrically relative to the center axis of the inner catheter **12**.

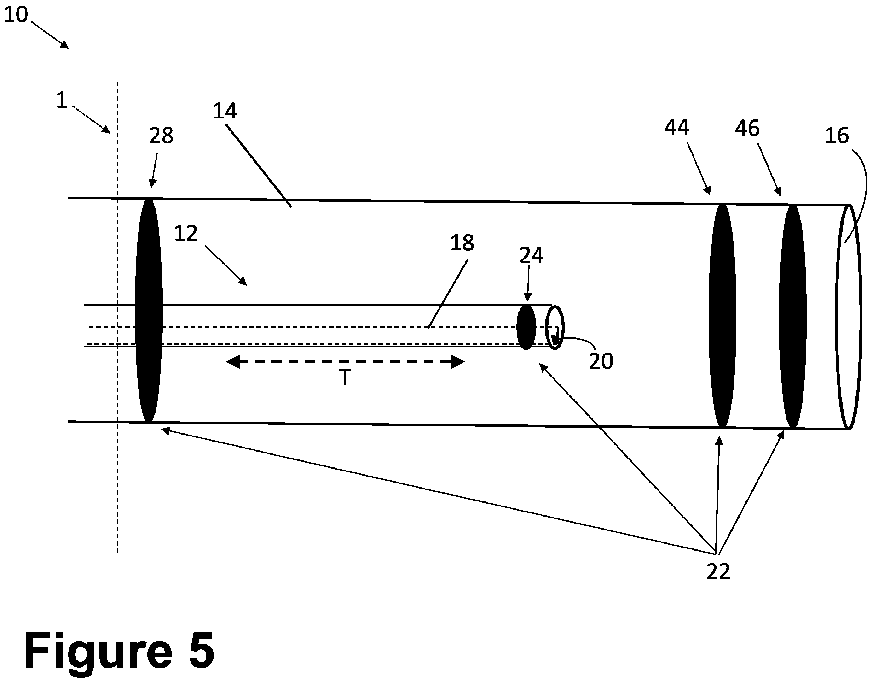

FIGURE 5 shows another embodiment of the device **10**. As shown in FIGURE 5, the proximity sensor **22** can include a first sensor **44** and a second sensor **46** disposed adjacent or close to the sheath opening **18**. The second sensor **46** is disposed closer to the sheath opening **18** than the first sensor **44**. When the catheter **12** (and thus the light output aperture **20**) is extended through the containment sheath **14** and out of the sheath opening **18**, this passage event is first detected by the first sensor **44**, and then the second sensor **46**. Conversely, when the extended light output aperture **20** is withdrawn into the containment sheath **14**, this passage event is first detected by the second sensor **46**, and then by the first sensor **44**. Advantageously, the accuracy of the proximity sensor **22** is improved.

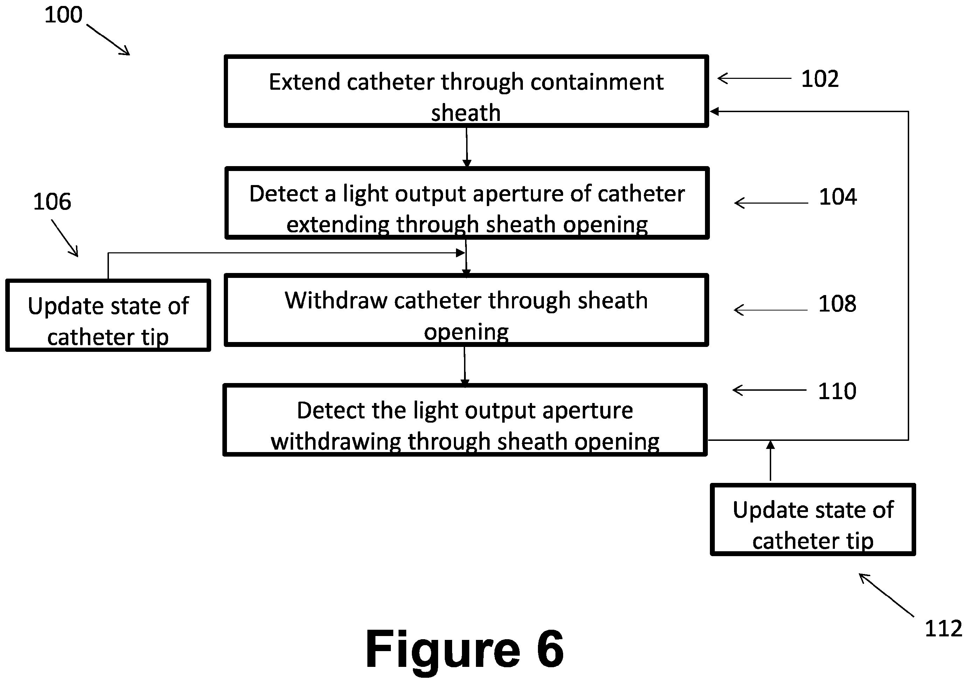

FIGURE 6 shows an example of a flowchart showing a vascular therapy method **100** using the device **10**. To being the method **100**, the device **10** is inserted into an artery or vein of the patient. Energy is supplied to the optical fiber(s) **18** via the laser coupler **34** for ablation of the lesion. At an operation **102**, the catheter **12** is extended through the containment sheath **14**. At an operation **104**, the safety sensor **22** is configured to detect a passage event of the light outlet aperture **20** extending beyond the sheath opening **18**. In some embodiments, in an optional operation **106**, the state machine **40** is updated to the outside state once the light outlet aperture **20** extends beyond the sheath opening **18**. At an operation **108**, the catheter **12** is withdrawn through the sheath opening **18** such that the light outlet aperture **20** is within the containment sheath **14**. At an operation **110**, the safety sensor **22** is configured to detect a passage event of the light outlet aperture **20** extending past the sheath opening **18** and into an interior of the containment sheath **14**. In some embodiments, in an optional operation **112**, the state machine **40** is updated to the inside state once the light outlet aperture **20** is detected to be within the containment sheath **14**. The method **100** can be performed iteratively until a treatment session is over.

The disclosure has been described with reference to the preferred embodiments. Modifications and alterations may occur to others upon reading and understanding the preceding detailed description. It is intended that the exemplary embodiment be construed as including all such modifications and alterations insofar as they come within the scope of the appended claims.

## Claims

1. A vascular therapy device (**10**), comprising:
a catheter (**12**) including at least one optical fiber (**18**) with a light output aperture (**20**);
a containment sheath (**14**) having a sheath opening (**18**) disposed at an end thereof, the catheter disposed inside the containment sheath and movable relative to the containment sheath to extend the light output aperture beyond the sheath opening such that the light output aperture is outside of the containment sheath;
wherein the device is configured to operate in a saline mode in which saline flows through the containment sheath (**14**) and in a contrast mode in which contrast medium flows through the containment sheath; and
**characterized in that** the device further comprises:
a safety sensor (**22**) configured to detect whether the light output aperture is in a safe zone inside the containment sheath; and
a device controller (**42**) configured to automatically turn off a laser operating to inject light into the at least one optical fiber (**18**) of the catheter (**12**) in response to a safety interlock condition in which the device is operating in contrast mode and the safety sensor (**22**) detects that the light output aperture (**20**) is not in the safe zone inside the containment sheath.

2. The device (**10**) of claim 1, wherein the safety sensor (**22**) includes:
a proximity sensor (**22**) configured to detect passage events in which the light output aperture (**20**) passes through the sheath opening (**18**); and
an electronic processor (**36**) configured to detect whether the light output aperture is in the safe zone based at least on the passage events detected by the proximity sensor.

3. The device (**10**) of claim 2, wherein the proximity sensor (**22**) includes:
an aperture marker (**24**) disposed on the catheter (**12**); and
a sheath opening marker (**26**) disposed on the containment sheath (**14**);
wherein one of the aperture marker and the sheath opening marker is a passive marker and the other of the aperture marker and the sheath opening marker is an active marker configured to detect proximity of the passive marker.

4. The device (**10**) of any one of claims 1 to 3, wherein the safety sensor (**22**) further includes:
a backside proximity sensor (**28**) configured to detect passage events in which the light output aperture (**20**) passes through an interior boundary of the safe zone wherein the interior boundary is located inside the containment sheath (**14**);
wherein the electronic processor (**36**) is configured to detect whether the light output aperture is in the safe zone further based on the passage events detected by the backside proximity sensor.

5. The device (**10**) of claim 1, wherein the safety sensor (**22**) includes:
a radiopaque aperture marker (**24**) disposed on the catheter (**12**);
a radiopaque sheath opening marker (**26**) disposed on the containment sheath (**14**); and
an electronic processor (**36**) configured to:
receive images from an interventional radiology imaging device (**38**) that depict the radiopaque aperture marker and the radiopaque sheath opening marker; and
detect whether the light output aperture is in the safe zone based on the received images.

6. The device (**10**) of anyone one of claims 1-5, wherein the electronic processor (**36**) is programmed to implement a state machine (**40**) by:
determining an inside state of the light output aperture (**20**) indicative of whether the light output aperture is inside of the containment sheath (**14**);
update the state machine from the inside state to an outside state after determining that the light output aperture is outside of the containment sheath;
determine whether the catheter (**12**) is in the saline mode or the contrast mode; and
cease supplying optical energy to the at least one optical fiber (**18**) when (i) the catheter is in the contrast mode and (ii) the state of the light output aperture is in the outside state.

7. The device (**10**) of claim 6, wherein the electronic processor (**36**) is further programmed to:
update the state machine (**40**) of the light output aperture (**20**) from the outside state to the inside state by determining whether the aperture marker (**24**) no longer extends past the sheath opening marker (**26**).

## Patentansprüche

1. Gefäßtherapievorrichtung (**10**), umfassend:
einen Katheter (**12**), der mindestens eine optische Faser (**18**) mit einer Lichtaustrittsöffnung (**20**) einschließt;
eine Schutzhülse (**14**), die eine an einem Ende davon angeordnete Hülsenöffnung (**18**) aufweist, wobei der Katheter innerhalb der Schutzhülse angeordnet und relativ zur Schutzhülse beweglich ist, um die Lichtaustrittsöffnung über die Hülsenöffnung hinaus zu verlängern, sodass sich die Lichtaustrittsöffnung außerhalb der Schutzhülse befindet;
wobei die Vorrichtung so konfiguriert ist, dass sie in einem Kochsalzmodus, in dem Kochsalzlösung durch die Schutzhülse (**14**) fließt, und in einem Kontrastmittelmodus, in dem Kontrastmittel durch die Schutzhülse fließt, arbeitet; und
**dadurch gekennzeichnet, dass** die Vorrichtung weiter Folgendes umfasst:
einen Sicherheitssensor (**22**), der so konfiguriert ist, dass er erkennt, ob sich die Lichtaustrittsöffnung in einem sicheren Bereich innerhalb der Schutzhülse befindet; und
eine Vorrichtungssteuereinheit (**42**), die so konfiguriert ist, dass sie einen Laser, der arbeitet, um Licht in die mindestens eine optische Faser (**18**) des Katheters (**12**) zu injizieren, automatisch als Reaktion auf eine Sicherheitsverriegelungsbedingung abschaltet, bei dem die Vorrichtung im Kontrastmodus arbeitet und der Sicherheitssensor (**22**) erkennt, dass sich die Lichtaustrittsöffnung (**20**) nicht im sicheren Bereich innerhalb der Schutzhülse befindet.

2. Vorrichtung (**10**) nach Anspruch 1, wobei der Sicherheitssensor (**22**) Folgendes einschließt:
einen Näherungssensor (**22**), der so konfiguriert ist, dass er Durchgangsereignisse erkennt, bei denen die Lichtaustrittsöffnung (**20**) die Hülsenöffnung (**18**) durchdringt; und
einen elektronischen Prozessor (**36**), der so konfiguriert ist, dass er mindestens auf Grundlage der vom Näherungssensor erkannten Durchgangsereignisse erkennt, ob sich die Lichtaustrittsöffnung im sicheren Bereich befindet.

3. Vorrichtung (**10**) nach Anspruch 2, wobei der Näherungssensor (**22**) Folgendes einschließt:
eine auf dem Katheter (**12**) angeordnete Öffnungsmarkierung (**24**); und
eine auf der Schutzhülse (**14**) angeordnete Hülsenöffnungsmarkierung (**26**);
wobei eine von der Öffnungsmarkierung und der Hülsenöffnungsmarkierung eine passive Markierung ist und die andere von der Öffnungsmarkierung und der Hülsenöffnungsmarkierung eine aktive Markierung ist, die so konfiguriert ist, dass sie die Nähe der passiven Markierung erkennt.

4. Vorrichtung (**10**) nach einem der Ansprüche 1 bis 3, wobei der Sicherheitssensor (**22**) weiter Folgendes einschließt:
einen rückseitigen Näherungssensor (**28**), der so konfiguriert ist, dass er Durchgangsereignisse erkennt, bei denen die Lichtaustrittsöffnung (**20**) eine innere Begrenzung des sicheren Bereichs durchdringt, wobei sich die innere Begrenzung innerhalb der Schutzhülse (**14**) befindet;
wobei der elektronische Prozessor (**36**) so konfiguriert ist, dass er weiter auf Grundlage der vom rückseitigen Näherungssensor erkannten Durchgangsereignisse erkennt, ob sich die Lichtaustrittsöffnung im sicheren Bereich befindet.

5. Vorrichtung (**10**) nach Anspruch 1, wobei der Sicherheitssensor (**22**) Folgendes einschließt:
eine auf dem Katheter (**12**) angeordnete röntgendichte Öffnungsmarkierung (**24**);
eine auf der Schutzhülse (**14**) angeordnete röntgendichte Hülsenöffnungsmarkierung (**26**); und
einen elektronischen Prozessor (**36**), der konfiguriert ist zum:
Empfangen von Bildern von einer radiologischen Eingriffsbildgebungsvorrichtung (**38**), die die röntgendichten Öffnungsmarkierung und die röntgendichten Hülsenöffnungsmarkierung darstellen; und
Erkennen auf Grundlage der empfangenen Bilder, ob sich die Lichtaustrittsöffnung im sicheren Bereich befindet.

6. Vorrichtung (**10**) nach einem der Ansprüche 1-5, wobei der elektronische Prozessor (**36**) so programmiert ist, dass er eine Zustandsmaschine (**40**) implementiert durch:
Bestimmen eines inneren Zustands der Lichtaustrittsöffnung (**20**), der angibt, ob sich die Lichtaustrittsöffnung innerhalb der Schutzhülse (**14**) befindet;
Aktualisieren der Zustandsmaschine vom inneren Zustand in einen äußeren Zustand, nachdem bestimmt wurde, dass sich die Lichtaustrittsöffnung außerhalb der Schutzhülse befindet;
Bestimmen, ob sich der Katheter (**12**) im Kochsalzmodus oder im Kontrastmodus befindet; und
Beenden der Zufuhr optischer Energie an die mindestens eine optische Faser (**18**), wenn (i) sich der Katheter im Kontrastmodus befindet und (ii) sich die Lichtaustrittsöffnung im äußeren Zustand befindet.

7. Vorrichtung (**10**) nach Anspruch 6,
wobei der elektronische Prozessor (**36**) weiter programmiert ist zum:
Aktualisieren der Zustandsmaschine (**40**) der Lichtaustrittsöffnung (**20**) vom äußeren Zustand in den inneren Zustand, indem bestimmt wird, ob die Öffnungsmarkierung (**24**) nicht mehr über die Hülsenöffnungsmarkierung (**26**) hinausragt.

## Revendications

1. Dispositif de thérapie vasculaire (**10**), comprenant :
un cathéter (**12**) incluant au moins une fibre optique (**18**) avec une ouverture de sortie de lumière (**20**) ;
une gaine de confinement (**14**) présentant un orifice de gaine (**18**) disposé à une extrémité de celle-ci, le cathéter étant disposé à l'intérieur de la gaine de confinement et mobile par rapport à la gaine de confinement pour étendre l'ouverture de sortie de lumière au-delà de l'orifice de gaine de sorte que l'ouverture de sortie de lumière se trouve à l'extérieur de la gaine de confinement ;
dans lequel le dispositif est configuré pour fonctionner en mode solution saline, dans lequel une solution saline circule à travers la gaine de confinement (**14**), et en mode contraste, dans lequel un produit de contraste circule à travers la gaine de confinement ; et
**caractérisé en ce que** le dispositif comprend en outre :
un capteur de sécurité (**22**) configuré pour détecter si l'ouverture de sortie de lumière se trouve dans une zone de sécurité à l'intérieur de la gaine de confinement ; et
un dispositif de commande de dispositif (**42**) configuré pour désactiver automatiquement un laser fonctionnant pour injecter de la lumière dans la ou les fibres optiques (**18**) du cathéter (**12**) en réponse à une condition d'interverrouillage de sécurité dans laquelle le dispositif fonctionne en mode contraste et le capteur de sécurité (**22**) détecte que l'ouverture de sortie de lumière (**20**) ne se trouve pas dans la zone de sécurité à l'intérieur de la gaine de confinement.

2. Dispositif (**10**) selon la revendication 1, dans lequel le capteur de sécurité (**22**) inclut :
un capteur de proximité (**22**) configuré pour détecter des événements de passage au cours desquels l'ouverture de sortie de lumière (**20**) traverse l'orifice de gaine (**18**) ; et
un processeur électronique (**36**) configuré pour détecter si l'ouverture de sortie de lumière se trouve dans la zone de sécurité sur la base au moins des événements de passage détectés par le capteur de proximité.

3. Dispositif (**10**) selon la revendication 2, dans lequel le capteur de proximité (**22**) inclut :
un marqueur d'ouverture (**24**) disposé sur le cathéter (**12**) ; et
un marqueur d'orifice de gaine (**26**) disposé sur la gaine de confinement (**14**) ;
dans lequel l'un du marqueur d'ouverture et du marqueur d'orifice de gaine est un marqueur passif et l'autre du marqueur d'ouverture et du marqueur d'orifice de gaine est un marqueur actif configuré pour détecter la proximité du marqueur passif.

4. Dispositif (**10**) selon l'une quelconque des revendications 1 à 3, dans lequel le capteur de sécurité (**22**) inclut en outre :
un capteur de proximité arrière (**28**) configuré pour détecter des événements de passage au cours desquels l'ouverture de sortie de lumière (**20**) traverse une limite intérieure de la zone de sécurité, dans lequel la limite intérieure est située à l'intérieur de la gaine de confinement (**14**) ;
dans lequel le processeur électronique (**36**) est configuré pour détecter si l'ouverture de sortie de lumière se trouve dans la zone de sécurité sur la base des événements de passage détectés par le capteur de proximité arrière.

5. Dispositif (**10**) selon la revendication 1, dans lequel le capteur de sécurité (22) inclut :
un marqueur d'ouverture radio-opaque (**24**) disposé sur le cathéter (**12**) ;
un marqueur d'orifice de gaine radio-opaque (**26**) disposé sur la gaine de confinement (**14**) ; et
un processeur électronique (**36**) configuré pour :
recevoir des images d'un dispositif d'imagerie de radiologie interventionnelle (**38**) qui représentent le marqueur d'ouverture radio-opaque et le marqueur d'orifice de gaine radio-opaque ; et
détecter si l'ouverture de sortie de lumière se trouve dans la zone de sécurité sur la base des images reçues.

6. Dispositif (**10**) selon l'une quelconque des revendications 1 à 5, dans lequel le processeur électronique (**36**) est programmé pour mettre en œuvre une machine à états (**40**) par :
la détermination d'un état intérieur de l'ouverture de sortie de lumière (20) indiquant si l'ouverture de sortie de lumière se trouve à l'intérieur de la gaine de confinement (14) ;
la mise à jour de la machine à états de l'état intérieur à un état extérieur après avoir déterminé que l'ouverture de sortie de lumière se trouve à l'extérieur de la gaine de confinement ;
la détermination si le cathéter (12) est en mode solution saline ou en mode contraste ; et
l'arrêt de la fourniture d'énergie optique à la ou aux fibres optiques (18) lorsque (i) le cathéter est en mode contraste et (ii) l'état de l'ouverture de sortie de lumière est à l'état extérieur.

7. Dispositif (**10**) selon la revendication 6,
dans lequel le processeur électronique (**36**) est en outre programmé pour :
mettre à jour la machine à états (**40**) de l'ouverture de sortie de lumière (**20**) de l'état extérieur à l'état intérieur par la détermination si le marqueur d'ouverture (**24**) ne s'étend plus au-delà du marqueur d'orifice de gaine (**26**).
